# EUROPÄISCHE PATENTSCHRIFT

(11) **EP 2 146 759 B1**
(45) Veröffentlichungstag und Bekanntmachung des Hinweises auf die Patenterteilung: **17.09.2014**
(21) Anmeldenummer: 08749054.6
(22) Anmeldetag: 23.04.2008
(51) Int. Cl.: A61M 1/00, A61M 27/00, A61F 13/00

(54) **FLACHDRAINAGE FÜR WUNDBEHANDLUNGEN**
FLAT DRAINAGE SYSTEM FOR WOUND TREATMENT
SYSTÈME DE DRAINAGE PLAT POUR LE TRAITEMENT DE PLAIES

(30) Priorität: 29.04.2007 DE 102007020740
(43) Veröffentlichungstag der Anmeldung: 27.01.2010
(73) Patentinhaber: ISKIA GmbH & Co. KG, 38829 Harsleben (DE)
(72) Erfinder: NEUBAUER, Norbert, 38820 Halberstadt (DE)
(74) Vertreter: Pfeiffer, Rolf-Gerd
(86) Internationale Anmeldenummer: PCT/EP2008/003251
(87) Internationale Veröffentlichungsnummer: WO 2008/131895

(56) Entgegenhaltungen:
- WO-A-01/89431
- WO-A-2005/123170
- US-A- 5 176 663
- US-A1- 2002 115 952
- US-A1- 2006 079 852

## Beschreibung

Die Erfindung betrifft eine Flachdrainage, die für verschiedene Formen von Wundbehandlungen verwendet werden kann, insbesondere jedoch zur Vakuumwundbehandlung von großen und tiefen Wunden konzipiert ist.

Es ist eine häufige Aufgabenstellung in der Medizin, Wundflüssigkeiten abzusaugen. Insbesondere ist bei tiefen, großen und dabei insbesondere von infizierten Oberflächenwunden die bislang übliche Praxis, in die Wunde eine Wundauflage, die nicht mit dem Gewebe verwächst, einzulegen. Auf diese Wundauflage bringt der Mediziner eine erste Lage Mull auf, in die dann von Hand ein Drainageschlauch, teilweise mehrfach gewunden, aufgelegt wird und dieser abermals mit einer zweiten Lage Mull abgedeckt und anschließend die gesamte Wundstelle mit einem Pflaster überklebt wird. Das Ende des Drainageschlauches wird dann mit einem Unterdruck beaufschlagt, wodurch die Wundflüssigkeit abgesaugt werden kann. Neben der langen Dauer der zur Verlegung vorstehend beschriebener Mittel benötigten Zeit, erfordert diese Vorgehensweise auch ein erhebliches Geschick des die Wunde versorgenden Mediziners, weil während der Wundversorgung alle separat eingelegten Vorrichtungsbestandteile zu fixieren sind, was häufig nicht durch eine Person allein bewerkstelligt werden kann.

Eine andere Art von Wundauflagen, die insbesondere für die Vakuumwundbehandlung entwickelt wurden, ist bspw. in DE 601 18 546 T2 beschrieben. Die dort beschriebene Wundauflage bedingt zum einen, einen relativ hohen Fertigungsaufwand und ist zum anderen nicht ohne weiteres an unterschiedliche Wundgrößen anpassbar. Um eine Vakuumbehandlung durchzuführen, bedarf diese Lösung zum anderen weiterer relativ kompliziert ausgeführter zusätzlicher Auflagen und in der Regel glockenartiger Abschlüsse der Wunde, an die ein externer Vakuumanschluss angebracht wird. Solche, die Hautoberfläche weit überragende Bauformen schränken die Bewegungsfreiheit des Patienten erheblich ein und erzeugen darüber hinaus unangenehme zusätzliche Druckbelastungen. Vorstehend skizzierte Behandlung derartiger Wunden unter Unterdruckbeaufschlagung ist ein seit vielen Jahren praktiziertes Verfahren, das die Heilung auch tiefer und großflächiger Wunden durch permanenten Wundreiz positiv beeinflusst. Diese Art der Vakuumwundbehandlung ist bspw. in DE 694 25 881 T3,

DE 692 29 940 T2 und DE 692 24 847 T3 ausführlich beschrieben, weshalb hier nur darauf verwiesen werden soll.

Darüber hinaus existiert eine Vielzahl weiterer Lösungen, die hier jedoch nur beispielhaft aufgeführt werden sollen, weil sie weiter entfernt liegende technische Lösungen betreffen. So ist aus US 6,695,824 B2 eine Wundverbandabdeckung für äußere flache Wunden bekannt, die aus zwei Schichten besteht, wobei die erste Schicht direkt auf die Wunde aufgelegt wird und die zweite Schicht eine nach außen wirkende Feuchtigkeitssperre aufweist. Zwischen den genannten Schichten sind mehrere schlauchartige Zuführleitungen vorgesehen, die eine Feuchtigkeitsversorgung der Wunde gewährleisten. Für den Einsatz im Rahmen einer Wundbehandlung tiefer, schlecht heilender Wunden unter Zuhilfenahme der Technik der Vakuumwundbehandlung ist vorstehend genannte Wundverbandabdeckung jedoch nicht konzipiert und auch nicht geeignet, weil eine vakuumdichte Herausführung der teilweise mehrfach vorgesehenen Schläuche aus dem Wundbereich praktisch unmöglich ist.

Das gleiche trifft für vielfältige andere Vorrichtungen zur Absaugung von Wundsekreten aus Körperhöhlen zu, wie z.B. in EP 1 764 127 A1, WO 01/89431 A1, US 2006/0079852 A1 und WO 2005/123170 A1 beschrieben.

Vorliegender Erfindung liegt die Aufgabe zugrunde, ein Drainagemittel anzugeben, welches ohne großes handwerkliches Geschick vom Operateur passgenau auf die betreffende Wunde, insbesondere eine große tiefe Wunde, welche zur Vakuumwundbehandlung vorgesehen ist, anpassbar ist, keiner aufwendigen Fixierungen bei der Verlegung im Wundbereich bedarf und insbesondere eine leichte Abdichtbarkeit im Rahmen einer Vakuumwundbehandlung ermöglicht. Zugleich soll die erfindungsgemäße Flachdrainage eine effektivere Drainagewirkung bewirken, als es nach dem Stand der Technik bekannte Drainagen ermöglichen. Insbesondere soll die vorgeschlagene Flachdrainage auch eine verbesserte und für den Patienten erträglichere Vakuumwundbehandlung von großen offenen Wunden ermöglichen und einen deutlich erhöhten Tragekomfort gewährleisten.

Die Aufgabe der Erfindung wird durch die kennzeichnenden Merkmale des Patentanspruchs 1 gelöst. Vorteilhafte Ausgestaltungen sind Gegenstand der nachgeordneten Ansprüche.

Die Erfindung soll nachstehend anhand von mehreren unterschiedlichen Ausführungsbeispielen näher dargestellt werden. Die dargestellten Figuren zeigen schematisch:
- Fig. 1:: ein Beispiel einer Flachdrainage im seitlichen Schnitt;
- Fig. 2:: ein Beispiel einer Flachdrainage im seitlichen Schnitt;
- Fig. 3:: ein nach Figur 1 einer Flachdrainage im seitlichen Schnitt
- Fig. 4:: eine Draufsicht auf ein Beispiel nach Fig. 1 mit einer ersten Schicht;
- Fig. 5:: optisch transparenten ein Beispiel einer Flachdrainage im seitlichen Schnitt;
- Fig. 6:: eine Draufsicht auf ein Beispiel nach Fig. 5;
- Fig. 7:: eine Draufsicht auf die Ausführugsfrom unter Einsatz überwiegend optisch transparenter Materialien für alle funktionswesentlichen Vorrichtungsbestandteile und
- Fig. 7a:: einen im Auslauf einer schwanzförmigen Verlängerung nach Fig. 7 vorzusehenden Abdichtkörper.

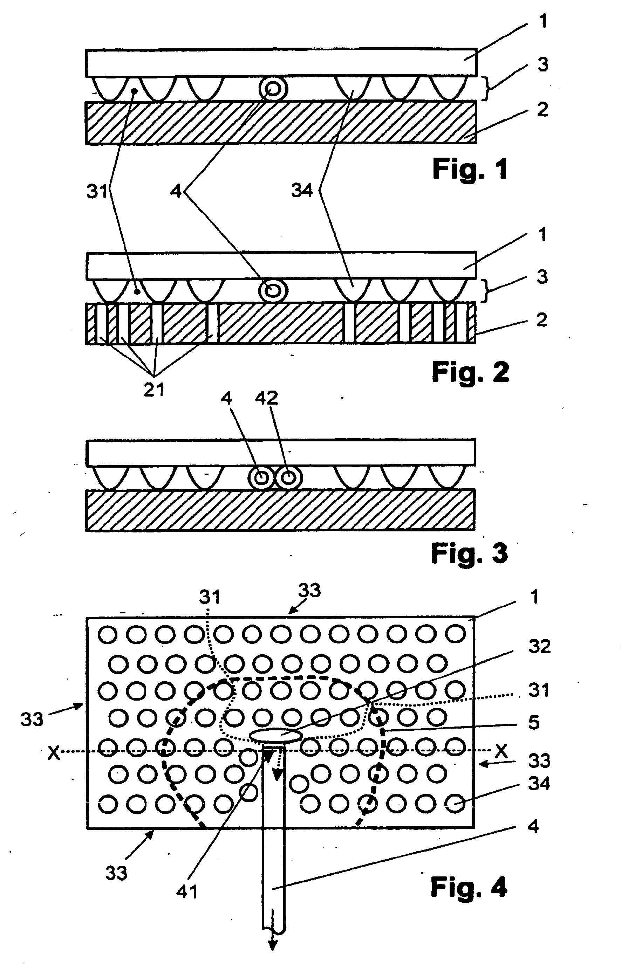

Ein Beispiel soll vom Grundprinzip her zunächst anhand der Figuren 1 und 2 näher beschrieben werden, die schematisch einen seitlichen Schnitt, senkrecht zu einer Ebene X-X nach Fig. 4 repräsentieren. Dabei sind aus genannten Figuren 1 und 2 die für die erfindungsgemäße Flachdrainage wesentlichsten Funktionsbestandteile ersichtlich, nämlich einer wundabseitigen ersten folienartigen flexiblen Schicht 1 und einer zweiten, der Wunde zugewandten flexiblen Schicht 2. Zwischen beiden genannten Schichten ist eine Ebene 3 vorgesehen, die beide Schichten 1; 2 voneinander, auch bei Unterdruckbeaufschlagung, derart beabstandet; dass zwischen diesen, in der Ebene 3 eingebrachten beabstandenden Mitteln 34 labyrinthartige Wege 31 gebildet sind und zwischen den Schichten 1;2 zumindest ein eingreifender Absaugschlauch 4 in einer ersten zu bevorzugenden seitlich eingebrachten Lage vorgesehen ist. Da die durch unregelmäßig eingebrachten beabstandenden Mitteln 34 gebildeten labyrinthartigen Wege 31 seitlich in der Ebene 3 nach außen münden, kann bei Anlegen eines Unterdrucks an den Absaugschlauch 4, bei hier nicht dargestellter sonstiger ganzflächiger Abdichtung der Vorrichtung gegen die Haut des Patienten, bereits bei dieser Ausführung eine hinreichende Absaugwirkung von Wundflüssigkeit erfolgen. Zur Verbesserung und vor allem Vergleichmäßigung der Absaugwirkung sind, wie in Figur 2 dargestellt, zumindest in der zweiten, der Wunde zugewandten Schicht 2 Perforationen/Durchbrüche 21 vorgesehen, die in die Ebene 3 führend, mit den labyrinthartigen Wegen 31 in Verbindung stehen. Je nach Art der Anbindung des Absaugschlauches 4 kann es zweckmäßig sein, die Perforationen oder Durchbrüche 21 mit größer werdendem Abstand von der Mündungsöffnung 41 dichter verteilt vorzusehen.

Figur 3 zeigt nur andeutungsweise eine Art der Erweiterungsmöglichkeit einer Ausfühtung am Beispiel nach Fig. 1, bei der eine weitere Zuleitung 42 neben der eigentlichen Drainageabsaugleitung 4 vorgesehen ist. Durch eine solche zusätzliche Leitung, die auch beliebig anders, z.B. als doppellumige Leitung ausgebildet sein kann, wäre es zusätzlich möglich, die Wunde durch Zufuhr von physiologischer Kochsalzlösung feucht zu halten und/oder, wenn gewünscht, Heilmittel in flüssiger Form zuzuführen. In einem solchen Fall ist es zweckmäßig, die zweite Schicht 2 aus einem stark hydrophilen Material, wie z.B. Alkantara-Leder, Polystyrol-, Polyestertextil oder Vergleichbarem zu fertigten, das die zugeführten Mittel löschblattartig über dem offenen Wundbereich verteilt. Mit dieser Ausführungsmöglichkeit soll lediglich die Multivalenz der Verwendbarkeit vorgeschlagener Flachdrainage für unterschiedliche Formen der Wundbehandlung verdeutlicht werden.

Der Hauptvorteil der vorgeschlagenen Flachdrainage liegt jedoch auf dem Gebiet der Vakuumwundbehandlung, worauf anhand der weiteren Figuren näher Bezug genommen wird.

Figur 4 zeigt eine Draufsicht auf ein Beispiel nach Fig. 1 mit einer optisch transparenten ersten Schicht 1. Insbesondere bei einer Ausführung der Erfindung und Beispiele gemäß den Figuren 1 bis 4 und 7 kann die in die Wunde eingelegte Flachdrainage inklusive des dann seitlich auf der gesunden Hautoberfläche außerhalb der Wunde abgeführten Schlauchs 4 durch eine hier nicht dargestellte luftundurchlässige weitere Klebefolie, die alle vorstehend genannten Baugruppen luftdicht gegen die Haut abgedichtet, eine Vakuumbehandlung, bspw. durch im Druck variierende Unterdruckbeaufschlagungen, durchgeführt werden. Diese zusätzlich zur Drainagewirkung durchführbare Vakuumbehandlung ist frei von den Nachteilen vergleichbarer bekannter Anordnungen. Eine solche Ausführung kann auf komplizierte Abdichtvorrichtungen, wie nach dem Stand der Technik bspw. aus WO 90/11795 üblich, vollständig verzichten.

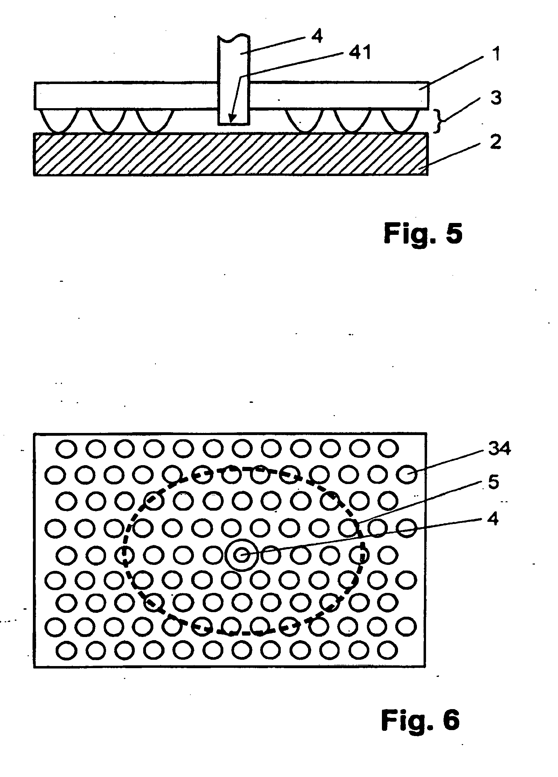

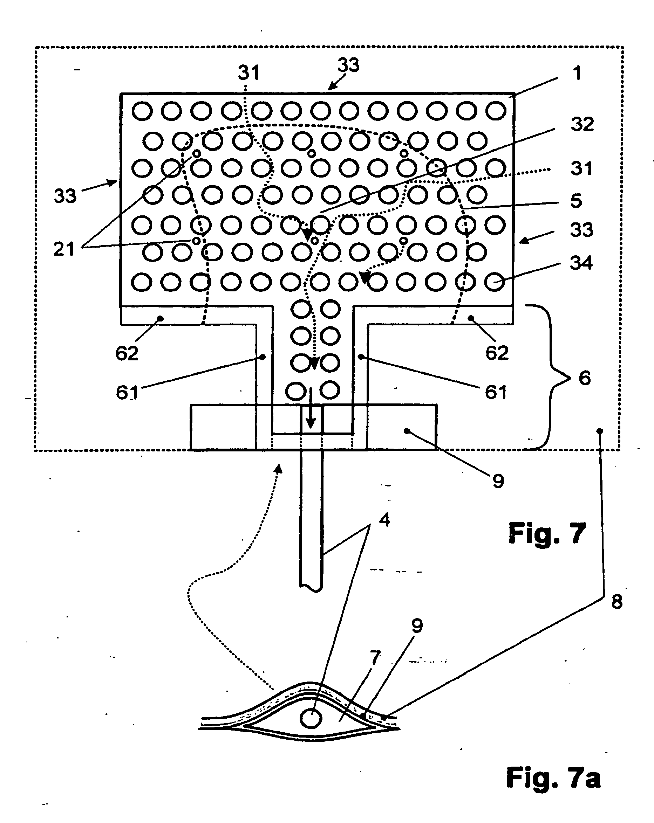

Durch Variation der Anzahl, Öffnungsgröße, Positionierung und gegenseitige Beabstandung der in Fig. 2 dargestellten Perforationen (die in gleicher Weise natürlich auch bei Ausführungen nach den Fig. 5 und 7 möglich sind), lässt sich zusätzlich die Stärke des auf die Wunde einwirkenden Vakuums voreinstellbar beeinflussen. Die hauptsächliche Unterdruckbeaufschlagung der Wunde erfolgt ansonsten über die seitlich offenen Randbereiche 33 der Flachdrainage, wie in Fig. 4 anhand beispielhafter Pfeile angedeutet. Bei einer Ausführung erfindungsgemäßer Flachdrainage nach Fig. 4 ist der Absaugschlauch 4 seitlich zwischen die Schichten 1; 2 eingelegt und mit diesen - verbunden, was ausführlicher anhand von Figur 7 beschrieben wird. Im Beispiel nach Fig. 4 erfolgt die Einbindung des Absaugschlauches 4 derart, dass seine Mündungsöffnung 41 bevorzugt etwa in der Flächenmitte der Flachdrainage zwischen der ersten und zweiten Schicht 1; 2 angeordnet ist (Gleiches gilt zu Fig. 5). Zusätzlich ist in dem Beispiel nach Fig. 4 der Mündungsöffnung 41 ein die direkte senkrechte Einströmung verhindernder Strömungsverteiler 32 vorgeordnet. Strömungs- und Unterdruckbeaufschlagungswege sind nur beispielhaft in Fig. 4 durch zwei punktierte Pfeilwege 31 dargestellt. Eine beispielhafte Zuschnittmöglichkeit der Flachdrainage ist in Fig. 4 durch eine strichlinierte Schnittlinie 5 angedeutet.

Sollte der Wundbehandler bereits aus dem Stand der Technik bekannte Ausrüstungsteile zur Vakuumwundbehandlung, die sich bspw. an sich unvorteilhafter glockenartiger Aufsätze bedienen, weiter verwenden wollen, kann er sich einer Flachdrainage gemäß der beispielhaften Darstellung nach Fig. 5 bedienen. Dort ist der Absaugschlauch 4 in etwa mittig durch die erste Schicht 1 hindurchgeführt und mit dieser abdichtend fest verbunden. Bei diesem Beispiel ragt der Absaugschlauch 4 bevorzugt in den Zwischenraum der Ebene 3 hinein und die ihm gegenüberliegende Schicht 2 weist in diesem korrespondierenden Flächenabschnitt keine Perforationen 21 auf. Selbst bei dieser an sich ungünstigeren Ausführung der Flachdrainage ist die erstmals durch vorliegende Erfindung geschaffene leichte Anpassbarkeit an die konkrete Wundfläche durch einfachen Zuschnitt entlang einer in Fig. 6 beispielhaft gezeigten Schnittlinie 5 gegeben.

Eine Draufsicht auf die Ausführungsform ist in Figur 7 dargestellt. In Variation zum vorstehend, anhand von Fig. 4 dargestellten Beispiel, zeichnet sich diese Ausführung dadurch aus, dass die Schichten 1; 2 und die sie beabstandende Ebene 3, beinhaltend die beabstandenden Mittel 34 (auf die weiter unten näher eingegangen wird), zumindest einseitig in Form einer schwanzförmigen Verlängerung 6 ausgebildet sind, in die stirnseitig der Absaugschlauch 4 umfangsmäßig abgedichtet eingreift. Dabei ist die flächenmäßige Ausbildung der Schichten 1; 2 und der sie beabstandenden Ebene 3 so groß ausgeführt, dass sie gemeinsam auf die tatsächliche Wundgröße und -form zuschneidbar sind (analog zur Ausführung nach Fig. 4), wobei die schwanzförmige Verlängerung 6 jedoch so lang ausgeführt ist, dass sie zumindest mit dem Teil, der den Absaugschlauch 4 dichtend aufnimmt, außerhalb des Wundbereichs anordenbar ist. Dabei sind die erste Schicht 1 und die zweite Schicht 2 zumindest im Bereich der schwanzförmigen Verlängerung 6 miteinander derart luftdicht verbunden, dass die sie voneinander ansonsten beabstandende Ebene 3 zum Rand hin stetig verjüngend ausgeführt ist.

Wird im Endbereich der schwanzförmigen Verlängerung 6 ein den Absaugschlauch 4 aufnehmender Abdichtkörper 7 vorgesehen, dessen äußeres Profil die zum Rand hin stetig verjüngende Ausformung der Ebene 3 in diesem Bereich vorgibt (siehe Figur 7a), kann die dichte Anbindung des Absaugschlauches 4 durch Verschweißung oder Verklebung problemlos realisiert werden. Damit die Absaugwirkung und Vakuumbeaufschlagung auf den eigentlichen Wundbereich auch nur dort gezielt erfolgt, sind die erste und zweite Schicht zusätzlich zur im Bereich der schwanzförmigen Verlängerung 6 miteinander vorgesehenen luftdichten Verbindung 61 auch in einem sich daran zur eigentlichen Flachdrainage unmittelbar anschließenden Bereich 62 der Flachdrainage miteinander luftdicht verbunden, wie es der hellgrau dargestellte Bereich in Fig. 7 beispielhaft zeigt. Diese Verbindung kann ebenfalls in einem einheitlich durchzuführenden Verschweißungs- oder Verklebungsprozess erfolgen. Die auch hier gegebene Zuschnittmöglichkeit der Flachdrainage ist wieder durch eine beispielhafte Schnittlinie 5 repräsentiert. Die Schicht 2 soll in diesem Beispiel im Bereich der schwanzförmigen Verlängerung 6 frei von Perforationen 21 sein. Von den im Wundbereich vorgesehenen Perforationen 21 sind in Fig. 7 nur einige beispielhaft gezeigt, um bereits hier anzudeuten, dass es besonders vorteilhaft ist, die ersten und zweite Schicht 1; 2 und die sie beabstandenden Mittel 34, zumindest im durch sie zu erfassenden Wundbereich, aus einem optisch transparenten Material zu fertigten. Dies erleichtert dem Wundbehandler, im Gegensatz zu allen bekannten Vorrichtungen nach dem Stand der Technik, die exakte gewünschte Positionierung der Flachdrainage im Wundbereich erheblich.

Die in Figur 7 dargestellte Ausführungsform lässt sich besonders vorteilhaft in einer offenen, tiefen und zur Vakuumwundbehandlung vorgesehenen Wunde, ggf. unter Unterfütterung hier nicht näher dargestellter oder auszuführender, die Wunde ausfüllender Mittel, wie Mull etc., verlegen und durch eine großflächige, alle genannten Baugruppen überdeckende luftdichte Klebefolie 8 (vgl. Fig. 7) gegen die Haut des Patienten abdichten. Die schwanzförmige Verlängerung 6 gewährleistet ferner, dass der Bereich des Schlauchanschlusses weit außerhalb der eigentlichen Wunde verlegbar ist und so der Schlauch selbst keinen störenden Druck auf die Wunde ausübt. Dadurch, dass das Ende der schwanzförmigen Verlängerung 6 außerhalb der Wunde auf der Haut des Patienten anordenbar ist, kann eine besonders einfache zusätzliche Vorfixierung dieses Ende (und damit Fixierung der Drainage selbst) vor Aufbringen genannter Folie 8 für den Operateur vermittels eines z.B. doppelseitig klebenden Pflasters 9 erreicht werden. Nicht nur durch den geschaffenen verlängerten äußeren Dichtweg entlang der die Bereiche 61 für die überdeckende Klebefolie, sondern auch durch die besondere Ausgestaltung des den Schlauch 4 aufnehmenden Abdichtkörpers 7 ist eine gesicherte luftdichte Überklebung der gesamten Flachdrainage als Voraussetzung zur Durchführung einer Vakuumwundbehandlung gewährleistet.

Für alle vorstehend beschriebenen Ausführungsmöglichkeiten der Erfindung gilt in gleicher Weise weiterhin, dass:
Die beabstandenden Mittel 34 wahlweise durch Noppen und/oder Stege gebildet können sein, die bevorzugt aus einem hydrophoben Material bestehen. Dabei können die durch Noppen und/oder Stege gebildeten beabstandenden Mitteln 34 vorteilhaft form- und/oder stoffschlüssiger Bestandteil der ersten und/oder zweiten Schicht 1; 2 sein. Insbesondere lassen sich Noppen durch elastische in sich jeweils geschlossene Luftpolster (vergleichbar mit von Verpackungsmitteln her bekannten Luftpolsterfolien, die zwischen einer ersten und zweiten flexiblen Schicht in einem einheitlichen Herstellungsprozess eingeschweißte, voneinander jedoch beabstandete noppenartige Luftpolsterbereiche beinhalten) bilden. Eine solche Ausführung, übertragen auf Fig. 7, zeigt die Vorteile dieser besonders zu bevorzugenden Ausführungsform im Hinblick auf die optische Transparenz, Flexibilität und gewichtsmäßige Leichtigkeit einer so hergestellten Flachdrainage. Im Rahmen der Erfindung sind die angesprochenen Noppen und oder Stege bevorzugt aus einem hydrophoben Material zu fertigen.
Es liegt aber ebenso im Rahmen der Erfindung, die die erste und zweite Schicht 1; 2 beabstandenden Mitteln 34 durch eine poröse Fleeceschicht, die ebenfalls optisch transparent herstellbar ist, oder durch eine poröse Textilschicht zu bilden.
Auch können die beabstandenden Mittel 34 durch ein locker aufgestreutes Schaumstoffgranulat mit geschlossenen Poren gebildet sein, wobei die Verbindung zu den benachbarten Schichten 1;2 bspw. durch Verklebung oder Verschweißung erfolgen kann. Auch solche einzelnen Schaumstoffgranulatpartikel sollten ebenfalls bevorzugt aus einem hydrophoben Material bestehen.

Besonders vorteilhaft, im Rahmen der beschrieben Ausführungsbeispiele, ist die Fertigung zumindest der zweiten Schicht aus einem an sich üblichen und kommerziell verfügbaren Wundauflagenmaterial, weil dieses neben den geforderten medizinischen Gesichtspunkten auch vorstehende Bedingungen der angegebenen Verarbeitungs- und Verwendungsmöglichkeiten von sich aus gewährleistet. In besonderer Ausgestaltung der Erfindung wurde hier dieses Wundauflagenmaterial zusätzlich mit einer, hier nicht darstellbaren, antimikrobiellen und/oder antiadhäsiven Beschichtung versehen.

Die erste Schicht 1 sollte bevorzugt wasserundurchlässig, aber luftdurchlässig ausgeführt sein.

Wenn Perforationen oder Durchbrüche 21 in der zweiten Schicht 2 vorgesehen sein sollen, was insbesondere bei flächenmäßig größeren Ausdehnungen der Flachdrainage anzuraten ist, sollten diese mit steigendem Abstand zur Absaugöffnung dichter verteilt angeordnet sein.

Die erfindungsgemäße Flachdrainage kann in unterschiedlichen Abmessungen vorkonfektioniert werden, wobei eine übliche flächenmäßige Ausdehnung (vgl. bspw. Fig. 4 und 7) in der Größenordnung von 5 · 6 cm liegt, was der überwiegenden Zahl gängiger Wundgrößen entspricht. Das Vorhalten anderer, insbesondere größerer Grundabmaße ist selbstverständlich problemlos möglich, da der passgenaue Zuschnitt zumindest im Rahmen des Wundbereiches bei jeder der Ausführungsformen ermöglicht ist.

Die Vorteile vorliegender Erfindung bestehen im Wesentlichen darin, dass:
- Alle beschriebenen Ausführungsformen passgenau auf jede Wundform zuschneidbar sind (vgl. beispielhafte Zuschnittlinien 5 in den Figuren 4, 6 und 7), ohne dass der Patient dadurch bei der Einpassung eine Belastung erfährt.
- Alle zur Ausbildung der Flachdrainage eingesetzten Einzelkomponenten flexibel, weich, elastisch und leicht ausführbar sind, so dass gegenüber bekannten Lösungen ein erhöhter Tragekomfort beim Patienten gegeben ist und vor allem keine störenden Druckbelastungen, insbesondere durch Schlauchenden im Wundbereich entstehen, die zu weiteren Komplikationen bei der Wundheilung führen können.
- Ausführungen nach den Figuren 1 bis 4 und 7 ermöglichen eine körpernahe Abdichtung aller Komponenten im Wesentlichen auf der Hautoberfläche, was dem Patienten eine leichtere Lageveränderung im Vergleich zu sonst bekannten Vakuumbehandlungsvorrichtungen ermöglicht.

Erfindungsgemäß vorgeschlagene Flachdrainage ist, wie eingangs erwähnt, insbesondere für die Durchführung einer wesentlich vereinfachten Vakuumwundbehandlung konzipiert. Verzichtet man jedoch auf die oben beschriebene luftdichte gesonderte Überklebung der Flachdrainage und anderer außerhalb dieser Erfindung liegenden Komponenten, kann die Flachdrainage, wahlweise abgewandelt durch die zu Fig. 3 beschriebenen Modifikationen, auch zu anderen Behandlungszwecken eingesetzt werden. Vorteilhafte Einsatzfälle dieser Flachdrainage in Verbindung mit wenigstens einer Zuleitung 42 können somit z.B. auch die Behandlung problematischer großflächiger und nässender Wunden darstellen. Bei deren Behandlung unter Einsatz sonst üblicher Wundauflagen, die dazu neigen ganz oder teilweise mit der Wunde zu verwachsen und daher einen häufigen Wechsel der Wundauflagen erforderlich machen, was den Heilungsprozess verlangsamt, könnte die Heilung unter Einsatz vorliegender Vorrichtung ebenfalls erheblich beschleunigt werden.

Auch liegt es im Rahmen der Erfindung, die vorgesehene erste Schicht 1 in spezieller Ausführung und Strukturierung analog zur Schicht 2 auszubilden. Dadurch könnte die Flachdrainage auch eingesetzt werden, wenn sie beidseitig mit Mulllagen oder sonstigen eine Wunde ausfüllenden Mitteln versehen wird.

## Patentansprüche

1. Flachdrainage für Wundbehandlungen, insbesondere zur Vakuumwundbehandlung von großen und tiefen Wunden unter gleichzeitigem Einsatz von üblichen luftdichten Wundabdeckungen, Drainageschläuchen und Vakuumabsaugvorrichtungen, wobei die Flachdrainage aus einer wundabseitigen ersten folienartigen flexiblen Schicht (1) und einer zweiten, der Wunde zugewandten flexiblen Schicht (2) besteht, wobei zwischen beiden genannten Schichten eine Ebene (3) vorgesehen ist, die beide Schichten (1; 2) voneinander, auch bei Unterdruckbeaufschlagung, derart beabstandet, dass zwischen eingebrachten beabstandenden Mitteln (34) labyrinthartige Wege (31) gebildet sind, welche **zumindest einseitig in Form einer schwanzförmigen Verlängerung (6) ausgebildet sind, in die stirnseitig ein Absaugschlauch (4) umfangsmäßig abgedichtet eingreift** und **die erste Schicht (1) und die zweite Schicht (2) zumindest im Bereich der schwanzförmigen Verlängerung (6) miteinander derart luftdicht verbunden sind,** dass die **sie voneinander ansonsten beabstandende Ebene (3) zum Rand hin stetig verjüngend ausgeführt ist und der** zwischen den Schichten (1; 2) zumindest eine eingreifende Absaugschlauch (4) derart vorgesehen ist, dass dieser in **der** schwanzförmigen Verlängerung (6) angeordnet ist, die so lang ausgeführt ist, dass sie zumindest mit dem Teil, der den Absaugschlauch (4) dichtend aufnimmt, außerhalb des Wundbereichs anordenbar ist, wobei der Absaugschlauch (4) selbst im Auslauf der schwanzförmigen Verlängerung (6) von einem dort vorgesehenen Abdichtkörper (7) aufgenommen ist, dessen äußeres Profil die zum Rand hin stetig verjüngende Ausformung der Ebene (3) in diesem Bereich vorgibt **und die flächenmäßige Ausbildung der Schichten (1: 2) und der sie beabstandenden Ebene (3) ansonsten so groß ausgeführt sind, dass sie gemeinsam auf die tatsächliche Wundgröße und -form zuschneidbar sind.**

2. Flachdrainage nach Anspruch 1, **dadurch gekennzeichnet, dass** die erste Schicht (1) und die zweite Schicht (2) zumindest im Bereich der schwanzförmigen Verlängerung (6) miteinander entlang von Seitenbereichen (61) luftdicht verbunden sind.

3. Flachdrainage nach Anspruch 1 und 2, **dadurch gekennzeichnet, dass** die erste Schicht (1) und die zweite Schicht (2) zusätzlich zur im Bereich der schwanzförmigen Verlängerung (6) miteinander vorgesehenen luftdichten Verbindung (61) auch in einem sich daran zur eigentlichen Flachdrainage unmittelbar anschließenden Bereich (62) der Flachdrainage miteinander luftdicht verbunden sind.

4. Flachdrainage nach Anspruch 1, **dadurch gekennzeichnet, dass** bei seitlicher Einbindung des Absaugschlauches (4) seiner Mündungsöffnung (41) ein die direkte senkrechte Einströmung verhindernder Strömungsverteiler (32) vorgeordnet ist.

5. Flachdrainage nach Anspruch 1, **dadurch gekennzeichnet, dass** ein Absaugschlauch (4) durch die erste Schicht (1) hindurchgeführt und mit dieser fest verbunden ist, wobei der Absaugschlauch (4) bevorzugt in den Zwischenraum der Ebene (3) hineinragt und die ihm gegenüberliegende Schicht (2) in diesem korrespondierenden Flächenabschnitt frei von Perforationen (21) ist.

6. Flachdrainage nach Anspruch 1, **dadurch gekennzeichnet, dass** die erste Schicht (1) wasserundurchlässig, aber luftdurchlässig ausgeführt ist.

7. Flachdrainage nach Anspruch 1, **dadurch gekennzeichnet, dass** zumindest die zweite Schicht (2) mit Perforationen (21) versehen ist, die in die Ebene (3) führend, mit den labyrinthartigen Wegen (31) in Verbindung stehen.

8. Flachdrainage nach Anspruch 1, **dadurch gekennzeichnet, dass** die zweite Schicht (2) mit Perforationen (21) versehen ist, die in die Ebene (3) führend, ausschließlich außerhalb des Bereichs der schwanzförmigen Verlängerung (6) mit den labyrinthartigen Wegen (31) in Verbindung stehen.

9. Flachdrainage nach Anspruch 7 oder 8, **dadurch gekennzeichnet, dass** genannte Perforationen (21) ungleichmäßig über die zweite Schicht (2) verteilt angeordnet sind.

10. Flachdrainage nach Anspruch 7 oder 8, **dadurch gekennzeichnet, dass** die Perforationen (21) oder Durchbrüche in der zweiten Schicht (2) mit größer werdendem Abstand von der Mündungsöffnung (41) dichter verteilt vorgesehen sind.

11. Flachdrainage nach Anspruch 1, **dadurch gekennzeichnet, dass** die beabstandenden Mittel (34) durch Noppen und/oder Stege gebildet sind, die bevorzugt aus einem hydrophoben Material bestehen.

12. Flachdrainage nach Anspruch 1 und 11, **dadurch gekennzeichnet, dass** die durch Noppen und/oder Stege gebildeten beabstandenden Mittel (34) form- und/oder stoffschlüssiger Bestandteil der ersten und/oder zweiten Schicht (1; 2) sind.

13. Flachdrainage nach Anspruch 12, **dadurch gekennzeichnet, dass** die Noppen durch elastische in sich jeweils geschlossene Luftpolster gebildet sind.

14. Flachdrainage nach Anspruch 1, **dadurch gekennzeichnet, dass** die beabstandenden Mittel (34) durch eine poröse Fleeceschicht gebildet sind, die bevorzugt aus einem hydrophoben Material besteht.

15. Flachdrainage nach Anspruch 1, **dadurch gekennzeichnet, dass** die beabstandenden Mittel (34) durch ein Schaumstoffgranulat mit geschlossenen Poren gebildet sind, wobei die einzelnen Schaumstoffgranulatpartikel bevorzugt aus einem hydrophoben Material bestehen.

16. Flachdrainage nach einem der vorstehenden Ansprüche, **dadurch gekennzeichnet, dass** die erste und zweite Schicht (1; 2) und die sie beabstandenden Mittel (34), zumindest im durch sie zu erfassenden Wundbereich, aus einem optisch transparenten Material gefertigt sind.

17. Flachdrainage nach einem der vorstehenden Ansprüche, **dadurch gekennzeichnet, dass** zumindest die zweite Schicht (2) aus einem Wundauflagematerial gefertigt ist.

18. Flachdrainage nach Anspruch 1 oder 17, **dadurch gekennzeichnet, dass** die zweite Schicht (2) zumindest wundseitig mit einer antimikrobiellen und/oder antiadhäsiven Beschichtung versehen ist.

19. Flachdrainage nach Anspruch 1, **dadurch gekennzeichnet, dass** die zweite Schicht (2) aus einem stark hydrophilen Material, wie Alkantara-Leder, Polystyrol-, Polyestertextil oder vergleichbarem gefertigt ist.

20. Flachdrainage nach Anspruch 19, **dadurch gekennzeichnet, dass** im Fall der Ausbildung der zweiten Schicht (2) aus hydrophilem Material zumindest eine zweite Zuleitung (42) für die Zuführungsmöglichkeit bspw. physiologischer Kochsalzlösung und/oder flüssigen Heilmitteln an diese Schicht (2) vorgesehen ist.

21. Flachdrainage nach Anspruch 20, **dadurch gekennzeichnet, dass** die zweite Zuleitung (42) parallel zum Absaugschlauch (4) geführt ist, oder mit diesem gemeinsam ummantelt oder verschweißt ist, oder in einem doppellumigen Schlauch vorgesehen ist.

## Claims

1. Flat drainage system for wound treatment, in particular for vacuum treatment of large and deep wounds, at the same time employing common airtight wound coverings, drainage tubes, and vacuum sucking devices, wherein the flat drainage comprises a first foil-like flexible layer (1) on the off-side of the wound, and a second flexible layer (2) opposite the wound, a plane (3) being provided between the two said layers which spaces apart the two layers (1; 2), even when a negative pressure is applied, in such a manner that labyrinth-like paths (31) are formed between the inserted means (34) which establish the space, said paths being designed at least on one side as tail-like extensions (6) into which, on the head side, a suction tube (4) engages, sealed on its circumference, and the first layer (1) and the second layer (2) are airtight connected to one another at least in the range of the tail-like extension (6) in such a manner that the plane (3) which otherwise is spacing apart said layers is designed as steadily tapering towards the border and in that said at least one engaging suction tube (4) between the layers (1; 2) is such provided that the same is arranged in the tail-like extension (6) which is executed such long that it may be arranged outside of the wound area at least with its portion which receives the suction tube (4) in a sealing manner, wherein the suction tube (4) itself is received by a sealing body (7) provided in the run-out of the tail-like extension (6), the outside contours of said sealing body (7) define the steadily tapering shape of the plane (3) in this range towards the border, and wherein the planar formation of the layers (1; 2) and the plane (3) spacing apart the former are executed such large that they may be cut up together to the proper size and shape of the wound.

2. Flat drainage system as claimed in claim 1, **characterized in that** said first layer (1) and said second layer (2) are air-tight connected to one another at least in the range of the tail-like extension (6) along the border ranges (61).

3. Flat drainage system as claimed in claim 1 and claim 2, **characterized in that** said first layer (1) and said second layer (2) are also air-tightly connected to one another in a range (62) of the flat drainage in addition to the air-tight connection (61) provided in the range of the tail-like extension (6), said range (62) being directly joining the proper flat drainage.

4. Flat drainage as claimed in claim 1, **characterized in that** a flow distributor (32) is pre-positioned to a mouth opening (41) of the suction tube (4), said flow distributor preventing a direct vertical inflow at a lateral insertion of the suction tube (4).

5. Flat drainage as claimed in claim 1, **characterized in that** a suction tube (4) is passed through the first layer (1) and is tightly secured to the same, wherein the suction tube (4) preferably projects into the interspace of the plane (3) and wherein the opposite located layer (2) in the corresponding area section is free of perorations (21).

6. Flat drainage as claimed in claim 1, **characterized in that** the first layer (1) is designed water tight, but permeable to air.

7. Flat drainage as claimed in claim 1, **characterized in that** at least the second layer (2) is provided with perforations (21) which, leading into the plane (3), are in connection to the labyrinth-like paths (31).

8. Flat drainage as claimed in claim 1, **characterized in that** the second layer (2) is provided with perforations (21) which, leading into the plane (3), are in connection to the labyrinth-like paths (31) exclusively outside of the range of the tail-like extension (6).

9. Flat drainage as claimed in claim 7 or 8, **characterized in that** said perforations (21) are arranged irregularly distributed over the second plane (2).

10. Flat drainage as claimed in claim 7 or 8, **characterized in that** the perforations (21) or the ruptures in the second layer (2) are provided increasingly more closely distributed to one another with an increasing distance from the mouth opening (41).

11. Flat drainage as claimed in claim 1, **characterized in that** the space establishing means (34) is formed by naps and/or webs, preferably consisting of a hydrophobic material.

12. Flat drainage as claimed in claim 1 and 11, **characterized in that** the space establishing means (34) formed by naps and/or webs are a formfitting and/or a material fitting component of the first and/or the second layer (!, 2).

13. Flat drainage as claimed in claim 12, **characterized in that** the naps are formed by elastic air cushions, each being closed in itself.

14. Flat drainage as claimed in claim 1, **characterized in that** the space establishing means (34) is formed by a porous fleece layer, preferably consisting of hydrophobic material.

15. Flat drainage as claimed in claim 1, **characterized in that** the space establishing means (34) is formed by a foamed material granule having closed pores, wherein the individual particles of the foamed material granule preferably consist of hydrophobic material.

16. Flat drainage as claimed in any of the preceding claims, **characterized in that** the first and the second layer (1; 2) and the space establishing means (34) establishing the space between the former are made of an optically transparent material, at least in the range of the wound to be covered by them.

17. Flat drainage as claimed in any of the preceding claims, **characterized in that** at least the second layer (2) is made of a wound covering material.

18. Flat drainage as claimed in claim 1 or 17, **characterized in that** the second layer (2) is provided at least wound-sided with an anti-microbial and/or an anti-adhesive coating.

19. Flat drainage as claimed in claim 1, **characterized in that** the second layer (2) is made of a strongly hydrophilic material such as alcantara leather, polystyrol textile, polyester textile or comparable ones.

20. Flat drainage as claimed in claim 19, **characterized in that**, provided that the second layer (2) is made of a hydrophilic material, at least a second feed line (42) for introducing, for example, physiological salt solution and/or liquid drugs is provided for said layer (2).

21. Flat drainage as claimed in claim 20, **characterized in that** the second feed line (42) is run in parallel to the suction tube (4) or is commonly sheazed with or welded together with said suction tube (4) or provided in a double-lumen tube.

## Revendications

1. Drainage plat pour traitement de plaies, pouvant être utilisé en particulier pour le traitement par pression négative de plaies étendues et profondes, en les couvrant en même temps de pansements étanches, et en appliquant de drains et de dispositifs d'aspiration; le drainage plat étant composé sur le côté opposé à la plaie, d'une première couche flexible (1) en forme de feuille et face à la plaie, d'une deuxième couche flexible (2), un plan (3) étant intercalé entre les deux couches susmentionnées afin de les maintenir (1,2) à une certaine distance l'une de l'autre, même lorsqu'une pression négative est appliquée, de sorte que des voies en labyrinthe (31) se forment entre les moyens (34) introduits à une certaine distance les uns des autres, au moins sur un côté, comme une prolongation (6) en forme de queue, dans laquelle, coté frontal, est introduit de manière étanche sur toute la circonférence, un tuyau d'aspiration et la première couche (1) et la deuxième couche (2) étant au moins dans la zone de la prolongation en forme de queue (6), liées l'une à l'autre de manière étanche de sorte que le plan (3) assurant le maintien à distance des deux couches l'une de l'autre, est configuré de manière de se rétrécir en direction du bord et que le tuyau d'aspiration (4) entrant entre les couches (1 ;2) est conçu de sorte qu'il soit disposé dans la prolongation (6) en forme de queue et étant si longue au moins avec la pièce recevant le tuyau d'aspiration (4) et le rendant étanche, peut être placé en dehors de la zone lésée et le tuyau d'aspiration (4) étant lui-même logé dans la sortie de la prolongation en forme de queue (6) et étant reçu à cet endroit par un corps d'étanchéité (7), du profil extérieur duquel dépend dans cette zone la forme diminuant vers l'extérieur du plan (3), la configuration des surfaces des couches (1,2) et du plan (3) assurant la distance entre les couches étant conçu si grande qu'il est possible de les découper ensemble jusqu'à ce que l'étendue et la forme de la plaie soient totalement couvertes.

2. Drainage plat suivant la revendication 1 est **caractérisé en ce que** la première couche (1) et la deuxième couche (2), au moins dans la zone de la prolongation en forme de queue (6), le long des zones latérales (61), sont liées l'une à l'autre de manière étanche.

3. Drainage plat suivant les revendications 1 et 2 est **caractérisé en ce qu'**en plus de la liaison étanche (61) prévue dans la zone de la prolongation en forme de queue (6), la première couche (1) et la deuxième couche (2) sont également étanchement liées l'une à l'autre dans la zone (62) située en aval du drainage plat proprement dit.

4. Drainage plat suivant la revendication 1 est **caractérisé en ce que** dans le cas de l'introduction latérale du tuyau d'aspiration flexible (4), en amont de l'orifice (41) du tuyau est monté un dispositif assurant la distribution du flux liquide (32) afin d'empêcher toute introduction perpendiculaire directe du liquide.

5. Drainage plat suivant la revendication 1 est caractérisé en ce qu'un tuyau flexible d'aspiration (4) solidairement lié à la première couche (1), traverse celle-ci pour sortir de préférence dans l'espace formé avec le plan (3), la couche opposée correspondante (2) étant est exempte de perforations (21).

6. Drainage plat suivant la revendication 1 est **caractérisé en ce que** la première couche (1) est imperméable à l'eau, mais étanche à l'air.

7. Drainage plat suivant la revendication 1 est caractérisé en ce qu'au moins la deuxième couche (2) est dotée de perforations (21) qui allant en direction du plan (3) est en contact avec les voies en labyrinthe (31).

8. Drainage plat suivant la revendication 1 est **caractérisé en ce que** la deuxième couche (2) dotée de perforations (21) qui amènent vers le plan (3), est uniquement en dehors de la zone de prolongation en forme de queue (6) liée aux voies en labyrinthe (31).

9. Drainage plat suivant les revendications 7 et 8 est **caractérisé en ce que** les dites perforations (21) sont distribuées de manière inégale sur la deuxième couche (2).

10. Drainage plat suivant les revendications 7 et 8 est **caractérisé en ce que** la densité des perforations ou des perçages dans la deuxième couche (2) grandit au fur et à mesure que la distance de l'orifice de sortie (41) augmente.

11. Drainage plat suivant la revendication 1 est **caractérisé en ce que** les moyens (34) assurant le maintien à distance, ont la forme de noppes ou de ponts et sont faits dans un matériau hydrophobe.

12. Drainage plat suivant les revendications 1 et 11 est **caractérisé en ce que** les moyens assurant le maintien à distance (34) sont formés par des noppes et ponts qui de par leur forme et/ou matériau, font partie de la première et/ou de la deuxième couche (1;2).

13. Drainage plat suivant la revendication 12 est **caractérisé en ce que** les noppes constituent des coussins d'air individuels élastiques et fermés.

14. Drainage plat suivant la revendication 1 est **caractérisé en ce que** les moyens assurant le maintien à distance (34) sont formés par une couche de fleece poreuse, de préférence faite dans un matériau hydrophobe.

15. Drainage plat suivant la revendication 1 est **caractérisé en ce que** les moyens assurant le maintien à distance (34) sont composés d'un granulat de mousse aux pores fermés, dont les particules de mousse doivent être faites de préférence dans un matériau hydrophobe.

16. Drainage plat suivant une des revendications précédentes est **caractérisé en ce que** la première et la deuxième couche (1; 2) ainsi que les moyens assurant le maintien à distance (34), sont réalisés dans un matériau optiquement transparent, au moins dans la zone de la plaie où le drainage intervient.

17. Drainage plat suivant une des revendications précédentes est **caractérisé en ce qu'**au moins la deuxième couche (2) est réalisée dans un matériau approprié de couvrir une plaie.

18. Drainage plat suivant les revendications et 1 ou 17 est **caractérisé en ce que** la deuxième couche (2) est couverte par un film antimicrobien et/ou anti-adhésif, au moins dans la zone face à la plaie.

19. Drainage plat suivant la revendication 1 est **caractérisé en ce que** la deuxième couche (2) est réalisée dans un matériau fortement hydrophile tel que cuir alcantara, textile au polystyrène, textile au polyester ou semblables.

20. Drainage plat suivant la revendication 19 est **caractérisé en ce que** dans le cas où la deuxième couche (2) est réalisée en matériau hydrophile, est prévue la mise en place d'au moins d'une deuxième possibilité d'approvisionnement (42) pour amener à cette couche (2) par ex. de l'eau physiologique et/ou des médicaments liquides.

21. Drainage plat suivant la revendication 20 est **caractérisé en ce que** la deuxième conduite d'amenée (42) soit située en parallèle au tuyau flexible d'aspiration (4), soit placée dans la même gaine, soit soudée au tuyau flexible, soit disposée dans un flexible à deux compartiments.
